# EUROPEAN PATENT SPECIFICATION

(11) **EP 3 909 760 B1**
(45) Date of publication and mention of the grant of the patent: **16.04.2025**
(21) Application number: 19908207.4
(22) Date of filing: 27.12.2019
(51) Int. Cl.: B32B 27/00, A61P 17/02, A61Q 19/00, A61K 9/70, A61L 15/24, A61L 15/26, A61L 15/30, A61L 15/58, A61K 8/02, A61K 8/81, A61K 8/87, A61K 8/90, A61K 47/32, A61K 47/34

(54) **LAMINATED ADHESIVE SHEET FOR SKIN**
LAMINIERTE KLEBEFOLIE FÜR HAUT
FEUILLE ADHÉSIVE STRATIFIÉE POUR LA PEAU

(30) Priority: 07.01.2019 JP 2019000433
(43) Date of publication of application: 17.11.2021
(73) Proprietor: Cosmed Pharmaceutical Co., Ltd., Kyoto-shi, Kyoto 601-8014 (JP)
(72) Inventor: QUAN, Ying-shu, Kyoto-shi, Kyoto 601-8014 (JP); HASEGAWA, Junya, Kyoto-shi, Kyoto 601-8014 (JP); AMAKAWA, Takashi, Kyoto-shi, Kyoto 601-8014 (JP); KAMIYAMA, Fumio, Kyoto-shi, Kyoto 601-8014 (JP)
(74) Representative: Ter Meer Steinmeister & Partner
(86) International application number: PCT/JP2019/051572
(87) International publication number: WO 2020/145210

(56) References cited:
- JP-A- 2003 192 580
- JP-A- 2007 159 603
- JP-A- 2008 037 798
- JP-A- 2008 037 798
- JP-A- 2009 512 495
- JP-A- 2014 047 167
- JP-A- H1 029 934
- US-A1- 2014 066 865
- US-B2- 8 864 727

## Description

### TECHNICAL FIELD

The present invention relates to a medical or cosmetic skin application sheet and relates in detail to an adhesion sheet including a laminate of an adhesive agent and a film.

### BACKGROUND ART

As various adhesive sheets for application to the skin, sheets having various properties have been developed and used according to the purpose of the application. The development promoted on one side is a product having high water-vapor permeability (Patent Document 1), and the development promoted on the other side is occlusive dressing therapy (ODT) for an increase of transdermal absorption. The ODT enables a lesion to be healed in a short time by applying ointment to an affected part, covering the affected part with a polyethylene or polyvinylidene chloride thin film, applying an adhesive tape for sealing the affected part to increase the transdermal absorption of the drug. For this purpose, a material having low water-vapor permeability is useful.

Recently developed is a technique for perforating stratum corneum with a fine needle, i.e., a microneedle to substantially improve drug permeation efficiency compared to an application method. A microneedle array refers to one including many microneedles collected on a substrate. A microneedle patch refers to an easy-to-use product formed by adding, to the microneedle array, an adhesion tape for attaching the microneedle array to the skin or a cover sheet for maintaining an aseptic condition until use.

The microneedle prepared using, as the material therefor, a substance such as carbohydrate that is dissolved in the body and lost by metabolism does not cause an accident even if the needle is broken and left in the skin. Instead, when the carbohydrate contains a drug, the microneedle inserted into the skin can be dissolved in the body to easily administer the drug intradermally or subdermally (Patent Document 2).

In particular, when the microneedle made from a biosoluble polymer material such as hyaluronic acid or collagen is inserted into the skin, the water in the skin is diffused into the microneedle to swell a needle portion inserted into the skin and thereafter dissolve the needle portion. This results in diffusing hyaluronic acid or collagen into the skin, leading to exertion of an anti-wrinkle action, or in diffusing into the skin a drug or a valuable substance preliminarily dissolved in the needle portion (Patent Documents 3 and 4).

Patent Document 5 is directed to a patch preparation including a support; and a pressure-sensitive adhesive layer containing an adherent polymer and a drug on one surface of the support.

In order to allow the microneedle to be swollen and dissolved in the skin, it is necessary to stably hold the microneedle on a surface of the skin during the swelling and the dissolution. Usually, an adhesive protection tape having a larger area than the area of the microneedle array is laid over the microneedle array to press the microneedle array against the surface of the skin and thus tightly hold the microneedle array by the adhesiveness of the protection tape.

The adhesive protection tape, however, heavily affects the solubility of the microneedle in the skin. The use of the protective adhesion tape having high water-vapor permeability allows evaporation of water entered from the skin into the microneedle array not to sufficiently swell the microneedle and thus to deaccelerate dissipation of a drug in the skin. Therefore, it is important for the tape to suppress the water evaporation from the skin.

As described above, it is important for the adhesion sheet for skin, or the protective adhesion sheet for microneedles to lower the water-vapor permeability. Using a low water-vapor-permeability support to satisfy the important condition increases toughness of a whole sheet not to allow the sheet to follow the motion of the skin when attached, thus generating a drawback of easily causing skin irritation.

### PRIOR ART DOCUMENTS

### PATENT DOCUMENTS

Patent Document 1: JP 2012-532903 W
Patent Document 2: JP 2003-238347 A
Patent Document 3: JP 2009-273872 A
Patent Document 4: JP 2010-029634 A
Patent Document 5: US 2014/0066865 A1

### SUMMARY OF THE INVENTION

### PROBLEM TO BE SOLVED BY THE INVENTION

A topical skin application sheet is required to have characteristics of having an adhesion effect for fixing the sheet to the skin for a certain period and having an ODT effect and sheet stretchability. An object of the present invention is to provide a topical skin application sheet having high affinity for skin and having an excellent ODT effect and excellent stretchability.

### MEANS FOR SOLVING THE PROBLEM

The inventors of the present invention have confirmed that a three-layer sheet attains both the ODT effect and the stretchability, the three-layer sheet including: an adhesion layer that has as high affinity for skin as an acrylic adhesive agent and is excellent in terms of attachment for a long period, but has high moisture permeability to have difficulty exhibiting the ODT effect; a rubber adhesive agent having low moisture permeability and excellent ODT; and a film serving as a laminate support and having as high stretchability as a urethane film. Thus, the invention has been made. The bond between the rubber adhesive agent and the acrylic adhesive agent is not always good, and the rubber adhesive agent and the acrylic adhesive agent are sometimes peeled from each other. Therefore, in order to further improve the physical properties, a polyurethane sheet has been interposed between the rubber adhesive agent-layer and the acrylic adhesive-agent layer to improve the lamination and thus succeed in inventing an adhesive sheet having excellent stretchability and affinity for skin and having an excellent ODT effect. Thus, the present invention has been completed.

The present invention provides a laminated adhesive sheet for skin as defined in claim 1. Advantageous embodiments of the invention are defined in dependent claims 2 to 7.

### EFFECTS OF THE INVENTION

The adhesive sheet according to the present invention is an adhesive sheet for skin having the following four-layer structure. The smaller the number is, the closer to the skin the layer is.
1. Adhesive-agent layer (skin adhesion layer) having high moisture permeability and thus having high affinity for skin Suitable examples include an acrylic adhesive agent, and a urethane adhesive agent. The sheet is made to have an excellent property of long-term bond to the skin by attaching the sheet to the skin as a surface to be contacted with the skin.
2. Intermediate-layer film (intermediate layer) having stretchability

A layer having a different polarity from the polarity of the skin adhesion layer and the protective adhesion layer is used to improve the mutual bond to the adhesion layers and thus form a further better adhesive sheet for skin. It is effective to laminate, as the layer, an intermediate-layer film (intermediate layer) having stretchability between both the layers. The intermediate layer is sandwiched between the skin adhesion layer and the protective adhesion layer.

Suitable examples include a urethane film and an EVA film. The intermediate layer is a film having high stretchability and having good bond to the skin adhesion layer and the protective adhesion layer. The intermediate layer desirably has a thickness of 5 to 100 µm.

### 3. Low moisture-permeability adhesive-agent layer (protective adhesion layer)

The layer has low moisture permeability and has a characteristic of having an excellent adhesion property without allowing water to escape from the skin, to accelerate the swelling or the dissolution of the microneedle array and also increase, in cases of attachment-type of medical goods or cosmetics, an effect of absorbing a valuable component to the skin. A rubber adhesive agent is used.

### 4. Support film (highly stretchable support) having stretchability

Suitable examples include a urethane film and an EVA film. The support is a film having high stretchability and having good bond to the protective adhesion layer.

### BRIEF DESCRIPTION OF THE DRAWING

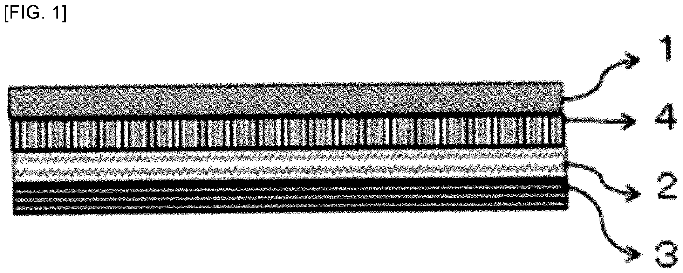

FIG. 1 is a sectional view illustrating a structure of an adhesive sheet according to the present invention. The drawing shows four layers, but the adhesive sheet has no intermediate layer in some cases.

### MODES FOR CARRYING OUT THE INVENTION

### 1. Skin adhesion layer

The skin adhesion layer constituting the adhesive sheet according to the present invention has high moisture permeability, is a surface contacted with the skin, and contains a drug or a valuable substance as necessary.

The adhesive agent contained in the skin adhesion layer is high moisture-permeability adhesive agent. It contains an acrylic adhesive agent or a urethane adhesive agent. Among these, the acrylic adhesive agent is the most preferable.

Examples of the acrylic adhesive agent include a homopolymer and a copolymer of an alkyl (meth)acrylate obtained from an aliphatic alcohol having 1 to 18 carbon atoms, particularly preferably 4 to 18 carbon atoms and (meth)acrylic acid; and a copolymer of an alkyl (meth)acrylate and another functional monomer.

Examples of the functional monomer include a hydroxy group-containing monomer, a carboxyl group-containing monomer, an amide group-containing monomer, and an amino-group containing monomer. As a copolymerizable monomer, for example, vinyl acetate, styrene, α-methylstyrene, vinyl chloride, acrylonitrile, ethylene, propylene, or butadiene can also be used. The adhesive agent preferably contains therein 50 mass% or more of an alkyl (meth)acrylate as a (co)polymerization component.

The urethane adhesive agent is an adhesive agent having a urethane group (-NCHCOO-), and is produced by reacting a polyester polyol or a polyether polyol with a polyvalent isocyanate or is made from a polyurethane polyol. The urethane adhesive agent made from a polyurethane polyol is preferable. The urethane adhesive agent can be, similarly to the acrylic adhesive agent, mixed with a plasticizer for further crosslinking, to be formed into an adhesive agent that is kind to the skin and has less adhesive residue.

Into the adhesive agents described above, a compounding agent such as a plasticizer, a filler, or an antiaging agent may be added as necessary.

The adhesive agents described above may contain therein a drug or a valuable substance as necessary.

The acrylic adhesive agent is usually prepared by solution polymerization of a desired monomer in the presence of a polymerization initiator. The form of polymerization, however, is not limited to the solution polymerization. In addition, the polymerization reaction conditions are appropriately selected mainly according to the type of a monomer.

The urethane adhesive agent can be prepared in conformity with the description in JP 2016-65238 A.

The skin adhesion layer is an adhesion layer that has a property of having a water-vapor permeation rate of 500 g/m^{2•}24 h or more and needs to have performance of absorbing water from the skin. The method for measuring the water-vapor permeation rate is described later. The skin adhesion layer desirably has a thickness of 20 to 80 µm.

### 2. Intermediate-layer film (intermediate layer) having stretchability

The intermediate layer is used for reinforcing the bonding between the skin adhesion layer and the protective adhesion layer. The intermediate layer needs to be a film having high stretchability and having good bond to the skin adhesion layer and the protective adhesion layer. Suitably, a urethane film or an EVA film is used. The intermediate-layer film needs to be stretched with weak force and unbroken. As dynamical parameters representing these characteristics, the intermediate layer desirably has a tensile modulus of elasticity of 0.1 or less and a breaking elongation of 100% or more. The unit of the tensile modulus of elasticity is 10⁴ kg/cm². The method for measuring mechanical properties of a film, such as tensile modulus of elasticity and breaking elongation is performed in conformity with ASTM D638. Specifically, a dumbbell-shaped film having a total length of 65 mm and a narrow parallel-portion length of 57 mm is set in a tensile tester (SHIMADZU CORPORATION) and pulled at a speed of 5 mm/min, and a stress-strain curve is obtained. The tensile modulus of elasticity is calculated from the tangent of the initial inclination of the curve, and the breaking elongation is measured from the elongation at breaking.

### 3. Low moisture-permeability adhesive-agent layer (protective adhesion layer)

The protective adhesion layer constituting the adhesive sheet according to the present invention is laminated with the skin adhesion layer to have a role in imparting a waterproof (low moisture permeability) function to the adhesive sheet. A rubber adhesive agent is used. Examples of the rubber adhesive agent include those obtained by adding, to 100 parts by mass of a rubber elastic body, 20 to 200 parts by mass of a tackifier, and an appropriate amount of a softener, a filler, or an antiaging agent as necessary. Examples of the rubber elastic body include natural rubber, a styrene-isoprene-styrene block copolymer, a styrene-butadiene-styrene block copolymer, a styrene-olefin-styrene block copolymer, polyisoprene, polybutene, polyisobutylene, and an ethylene-vinyl acetate copolymer. Examples of the tackifier include a rosin resin, a polyterpene resin, a coumarone-indene resin, a petroleum resin, and a terpene-phenol resin. Examples of the softer include liquid polybutene, mineral oil, lanolin, liquid polyisoprene, and liquid polyacrylate. Examples of the filler include titanium oxide. Examples of the antiaging agent include butylhydroxytoluene.

Into the adhesive agents described above, a compounding agent such as a plasticizer, a filler, or an antiaging agent may be added as necessary. For example, it is well known that the styrene-isoprene-styrene block copolymer itself does not become an adhesive agent and the styrene-isoprene-styrene block copolymer is mixed with a tackifier or an oil component to form an adhesive agent. As the oil component, liquid paraffin is general, but olive oil, jojoba oil or squalane can be used.

The protective adhesion layer desirably has a thickness of 5 to 200 µm.

### 4. Highly stretchable support

The property required of the highly stretchable support is equal to the property required of the intermediate-layer film, and a same film as the intermediate-layer film can be used as the highly stretchable support. Specifically, a urethane film, an EVA film, a urethane non-woven fabric or a polyester non-woven fabric are used. Adjusting the protective adhesion layer component makes it possible to extremely reduce the adhesiveness. Such a protective adhesion layer can play a role of the highly stretchable support.

The highly stretchable support has a thickness of usually 3 to 100 µm, preferably 5 to 60 µm from the viewpoint of softness.

The laminated adhesive sheet for skin of the invention has a value of the water-vapor permeation rate of 200 g/cm^{2•}24 h or less, preferably 100 g/cm^{2•}24 h or less. In addition, the laminated adhesive sheet for skin desirably has a tensile modulus of elasticity of 30 N or less.

### Release sheet

The adhesive sheet according to the present invention may include a release sheet on a surface of the skin adhesion layer for protection of adhesion force on the surface of the skin adhesion layer. The release sheet is removed from the adhesive sheet before application to the skin. As the release sheet, any agent usually used for an adhesive preparation is usable without limitation, and examples include a silicone resin-applied polyester film.

### Drug and valuable substance

As the drug and the valuable substance contained in the skin adhesion layer, any drug or valuable substance is acceptable as long as it can dermally permeate a biological membrane.

Examples of the drug include an antipyretic and anti-inflammation analgesic, an antiepileptic drug, an antipsychotic drug, an antidepressant and anti-anxiety drug, an antiparkinsonian drug, an antimanic drug, an antidementia drug, a sleeping drug, a sedative drug, an antispasm drug, a muscle relaxant, an autonomic drug, a cerebral circulation and metabolism improver, a cardiotonic drug, an antianginal drug, a drug for hypertension and arrhythmias, a vasodilator, a depressor, a vasopressor, a diuretic, a respiratory stimulant, an antitussive and expectorant drug, a bronchodilator, an asthma and nasal allergy medication, a bronchial dilator, a cold medication, an antiemetic, an antacid, and an anti-ulcerogenic drug.

The product according to the present invention has high affinity for a curve of the skin, and examples of the drug that contains an antipyretic and anti-inflammation analgesic and is suitable for application to a curved site such as an elbow include indomethacin, salicylic acid, glycol salicylate, aspirin, acetaminophen, diclofenac sodium, ibuprofen, sulindac, naproxen, and ketoprofen.

Examples of a component that can be blended as a cosmetic valuable substance include a whitening component, an anti-wrinkle component, an anti-inflammatory component, a blood circulation promoting component, an antibacterial component, an anti-itching component, various vitamins and derivatives thereof, an antioxidant component, an anti-allergy component, and a perfume.

Examples of the whitening component include, but are not particularly limited to, vitamin C derivatives such as an ascorbic acid phosphate magnesium salt, ascorbic acid glucoside and salts and acylated derivatives thereof, ethyl ascorbic acid, and ascorbyl palmitate; α-arbutin, β-arbutin, kojic acid, placenta extract, cysteine, glutathione, ellagic acid, rucinol, tranexamic acid, baicalein, and adenosine and sodium phosphate salts thereof; astaxanthin, deer horn shape Ganoderma lucidum, oil-soluble licorice, lavender, Zingiber Aromaticus, burnet, resveratrol, Ganoderma lucidum, and extract thereof; astaxanthin; and tinctures and components contained therein.

Examples of the anti-wrinkle component include, but are not particularly limited to, retinoids such as retinol, retinoic acid, retinol acetate, and retinol palmitate; α-hydroxy acids such as citric acid, fruit acid, glycolic acid, and lactic acid; α-hydroxy acid cholesterol, a rutin derivative, N-methylserine, elastin, collagen, sericin, Centella asiatica extract, and Scutellaria baicalensis root extract.

Examples of the anti-inflammatory component include, but are not particularly limited to, glycyrrhetinic acid, glycyrrhetinic acid 2K, allantoin, epsilon-aminocaproic acid, azulene, shikonin, tranexamic acid; Coptis japonica, licorice, Terminalia, yarrow, Lithospermum erythrorhizon, comfrey, aloe, butcher's broom, Horse-chestnut, peach leaf, loquat leaf, and extract thereof; and tinctures and components contained therein.

Examples of the blood circulation promoting component include, but are not particularly limited to, vitamin E, nicotinic acid, nicotinic acid amide, benzyl nicotinate, nicomol, caffeine, capsaicin, nonanoic acid vanillylamide, shogaol, and gingerol.

Examples of the antibacterial component include, but are not particularly limited to, cationic surfactants such as isopropyl methylphenol, triclosan, triclocarban, trichloro-hydroxyphenol, halocarban, benzalkonium chloride, and benzethonium chloride; a photosensitizer, zinc oxide, titanium oxide, chitin, chitosan, hinokitiol, and anise.

Examples of the anti-itching component include, but are not particularly limited to, diphenhydramine hydrochloride, chlorpheniramine maleate, crotamiton, glycyrrhizic acids, menthol, camphor, rosemary oil, capsaicin, nonanoic acid vanillylamide, and dibucaine.

Examples of the vitamins and derivatives thereof include, but are not particularly limited to, oil-soluble vitamins such as vitamin A oil, liver oil, retinol acetate, retinol palmitate, retinol, dehydroretinol, vitamin A₃, retinoic acid, vitamin D, vitamin D₂ (ergocalciferol), vitamin D₃ (cholecalciferol), a vitamin derivative, vitamin E (tocopherol), dl-α-tocopherol acetate, dl-α-tocopherol, tocopherol butyrate, tocopherol nicotinate, nicotinic acid benzyl ester, natural vitamin E, vitamin K, and vitamin U. Examples of the vitamins and derivatives thereof include, but are not particularly limited to, water-soluble vitamins such as vitamin B₁ (thiamine), vitamin B₂ (riboflavin tetrabutyrate), vitamin B₆ (fatty acid esters such as pyridoxine dicaprylate and pyridoxine dipalmitate), vitamin B₁₂ (cobalamin), vitamin B₁₃, vitamin B₁₄, and vitamin B₁₅ (pangamic acid) .

Examples of the antioxidant component include, but are not particularly limited to, polyphenols such as anthocyanin, catechin, green tea polyphenol, and apple polyphenol; carotenoids such as ascorbic acid, sodium ascorbate, sodium ascorbyl sulfate, β-carotene, and astaxanthin; β-diketones such as tocopherols, tocopherol acetate, natural vitamin E, tocomonoenol, tocotrienol, and curcumin; lignans such as sesamin and sesamolin; and phenols such as eugenol.

Examples of the anti-allergy component include, but are not particularly limited to, glycyrrhizin derivatives such as glycyrrhetinic acid and glycyrrhetinic acid 2K; licorice, chlorella, comfrey, moutan bark, small-leaved linden, Isodon herb, sage, Japanese basil, artemisia, and extract thereof; and tinctures and components contained therein.

Examples of the perfume include musuk, civet, thyme oil, and cinnamon bark oil.

### Method for producing adhesive sheet

One exemplary method for producing the adhesive sheet according to the present invention includes applying an ethyl acetate solution of an acrylic adhesive agent onto release PET and drying the solution to form a skin adhesion layer and then laminating the skin adhesion layer on one side of an intermediate layer. As the intermediate layer, a urethane film having a thickness of about 5 µm is used. A protective adhesion layer is formed by applying a rubber adhesive-agent solution onto release PET and drying the solution. Further the protective adhesion layer is laminated on a same urethane film as the one used for the intermediate layer to be backed by a highly stretchable support. Thereafter, a surface of the protective adhesion layer obtained by peeling the release PET is laminated on the other side of the intermediate layer to form a product. Adhesive sheets having other structures can also be produced by a similar procedure.

### Water-vapor permeation rate

The water-vapor permeation rate is measured in conformity with JIS Z 0208 [Testing Methods for Determination of the Water Vapor Transmission Rate of Moisture-Proof Packaging Materials (Dish Method) ].

### Tensile modulus of elasticity

A tensile tester (compact bench tester EZ Test EZ-SX) manufactured by SHIMADZU CORPORATION is used. A dumbbell piece having a width of 10 mm and a length of 50 mm and having a wider portion on both ends thereof is used as a sample. The sample is pulled at a tensile speed of 10 mm/min, and the modulus of elasticity is obtained from the inclination of a linear portion of the stress-strain curve. The modulus of elasticity in the present invention is a value of stress (N) at a strain of 10 mm.

### EXAMPLES

Hereinafter, the present invention is described with reference to examples. The present invention, however, is not limited to the examples.

### Example 1

An ethyl acetate solution of a HiPAS adhesive agent (acrylic adhesive agent, manufactured by CosMED Pharmaceutical Co. Ltd.) was applied onto a PET release film and dried to give a film (skin adhesion layer) having a thickness of 50 µm. The obtained adhesive-agent film was laminated on a urethane sheet (product manufactured by KYOWA INDUSTRY Co., Ltd., thickness: 50 µm) (intermediate layer) to form a laminate. Separately, 100 parts by mass of a styrene/isoprene triblock copolymer (SIS) (styrene content: 30 mass%, manufactured by Kraton Polymers Japan Ltd.) and 100 parts by mass of liquid paraffin were dissolved in toluene, applied onto a PET release film and dried to give a film (protective adhesion layer) having a thickness of 80 µm. The obtained adhesive-agent film was laminated on a urethane sheet (thickness: 7 µm) (highly stretchable support) to form a laminate. The two laminates were laminated together, with a surface of the protective adhesion layer opposite to a surface on a surface of the intermediate layer, to form a four-layer laminated adhesion sheet.

### Reference Example 2

A three-layer laminated adhesion sheet was produced similarly to Example 1 except that a urethane sheet was not used as the intermediate layer.

### Example 3

A four-layer laminated adhesion sheet was produced similarly to Example 1 except that 20 mass% of octyldodecyl lactate relative to the HiPAS adhesive agent was added to the skin adhesion layer and the thickness of support urethane sheet was changed to 10 µm.

### Example 4

A four-layer laminated adhesion sheet was produced similarly to Example 1 except that an EVA (ethylene-vinyl acetate copolymer resin) film was used as the intermediate layer and the thickness of the support urethane sheet was changed to 10 µm.

### Example 5

A four-layer laminated adhesion sheet was produced similarly to Example 1 except that 20 mass% of octyldodecyl lactate and 1.0 mass% of ascorbyl palmitate (RIKEN VITAMIN Co., Ltd.) relative to the HiPAS adhesive agent were added to the skin adhesion layer and in place of liquid paraffin, jojoba oil (NIKKOL jojoba oil S, Nikko Chemicals Co., Ltd.) was added to the protective adhesion layer.

### Example 6

A four-layer laminated adhesion sheet was produced similarly to Example 1 except that a urethane non-woven fabric was used as the intermediate layer and the thickness of the support urethane sheet was changed to 50 µm.

### Example 7

A four-layer laminated adhesion sheet was produced similarly to Example 1 except that the protective adhesion layer was formed using polyisobutylene (PIB) and liquid paraffin.

### Comparative Example 1

A four-layer laminated adhesion sheet was produced similarly to Example 1 except that a PET film was used as the intermediate layer.

### Comparative Example 2

A four-layer laminated adhesion sheet was produced similarly to Example 1 except that in place of the SIS adhesive agent, a HiPAS adhesive agent was used as the protective adhesion layer and the thickness of the protective adhesion layer was changed to 50 µm.

**[Table 1]**

| | Skin adhesion layer Thickness (µm) | Intermediate layer Thickness (µm) | Protective adhesion layer Thickness (µm) | Support Thickness (µm) |
|---|---|---|---|---|
| Example 1 | HiPAS adhesive agent (50) | Urethane sheet (50) | SIS + liquid paraffin (80) | Urethane sheet (7) |
| Ref. Example 2 | Same as above | None | Same as above | Same as above |
| Example 3 | HiPAS adhesive agent + octyldodecyl lactate (20 mass% relative to adhesive agent) (50) | Urethane sheet (50) | SIS + liquid paraffin (80) | Urethane sheet (10) |
| Example 4 | HiPAS adhesive agent (50) | EVA film (Japan Polyethylene Corporation) (20) | SIS + liquid paraffin (80) | Urethane sheet (10) |
| Example 5 | HiPAS adhesive agent octyldodecyl lactate (20 mass% relative to adhesive agent) | Urethane sheet (50) | SIS + jojoba oil (30 mass% relative to SIS) (80) | Urethane sheet (7) |
| | Ascorbyl palmitate (1 mass% relative to adhesive agent) (50) | | | |
| Example 6 | HiPAS adhesive agent (50) | Urethane non-woven fabric (50) | SIS + liquid paraffin (80) | Urethane sheet (50) |
| Example 7 | HiPAS adhesive agent (50) | Urethane sheet (50) | Polyisobutylene + liquid paraffin (50 mass% relative to PIB) (80) | Urethane sheet (7) |
| Comparative Example 1 | HiPAS adhesive agent (50) | PET film (50) | SIS + liquid paraffin (80) | Urethane sheet (7) |
| Comparative Example 2 | HiPAS adhesive agent (50) | Urethane sheet (50) | HiPAS adhesive agent (50) | Urethane sheet (7) |

Table 1 shows the features of the produced laminated adhesive-agent sheets. The obtained laminated adhesive-agent sheets were measured for the water-vapor permeation rate, the tensile modulus of elasticity, and the breaking elongation.

The water-vapor permeation rate was measured in conformity with JIS Z 0208 [Testing Methods for Determination of the Water Vapor Transmission Rate of Moisture-Proof Packaging Materials (Dish Method) ].

As regards the tensile modulus of elasticity, the laminated adhesive-agent sheets were each cut in a dumbbell shape having a width of 10 mm and a length of 50 mm and having a wider portion on both ends thereof, and the dumbbell-shaped sample was set in a tensile tester (manufactured by SHIMADZU CORPORATION, compact bench tester EZ test EZ-SX) and pulled at a speed of 10 mm/min, to obtain a stress-strain curve. The tensile modulus of elasticity was calculated from the inclination of an initial linear portion of the curve.

Table 2 shows the measurement results (single measurement).

**[Table 2]**

| | Water-vapor permeation rate g/m²•h | Tensile modulus of elasticity N |
|---|---|---|
| Example 1 | 53.4 | 3.6 |
| Ref. Example 2 | 70.2 | 3.5 |
| Example 3 | 47.5 | 3.8 |
| Example 4 | 55.8 | 21.0 |
| Example 5 | 49.3 | 3.4 |
| Example 6 | 51.2 | 3.2 |
| Example 7 | 48.4 | 3.4 |
| Comparative Example 1 | 35.3 | 143 |
| Comparative Example 2 | 318.7 | 3.4 |

### DESCRIPTION OF REFERENCE SYMBOLS

- 1: skin adhesion layer
- 2: protective adhesion layer
- 3: highly stretchable support
- 4: intermediate layer

## Claims

1. A laminated adhesive sheet for skin comprising a skin adhesion layer, an intermediate layer, a protective adhesion layer, and a highly stretchable support laminated in this order,
wherein the skin adhesion layer contains an acrylic adhesive agent or a urethane adhesive agent,
wherein the highly stretchable support is made of a polyurethane film, an ethylene vinyl acetate film, a urethane non-woven fabric, or a polyester non-woven fabric,
wherein the protective adhesion layer contains a rubber adhesive agent,
wherein the intermediate layer is made of a polyurethane film or an ethylene vinyl acetate film,
the laminated adhesive sheet having a water-vapor permeation rate of 200 g/m^{2•}24 h or less.

2. The laminated adhesive sheet according to claim 1, having a tensile modulus of elasticity of 30 N or less.

3. The laminated adhesive sheet according to claim 1 or 2, wherein the skin adhesion layer has a thickness of 20 to 80 µm.

4. The laminated adhesive sheet according to any one of claims 1 to 3, wherein the highly stretchable support has a thickness of 5 to 60 µm.

5. The laminated adhesive sheet according to any one of claims 1 to 4, comprising a release sheet on a surface of the skin adhesion layer.

6. The laminated adhesive sheet according to any one of claims 1 to 5, wherein the skin adhesion layer contains a drug or a valuable substance,
wherein the valuable substance is selected from the group consisting of a whitening component, an anti-wrinkle component, an anti-inflammatory component, a blood circulation promoting component, an antibacterial component, an anti-itching component, various vitamins and derivatives thereof, an antioxidant component, an anti-allergy component, and a perfume.

7. The laminated adhesive sheet according to claim 6, wherein the drug is selected from the group consisting of an antipyretic and anti-inflammation analgesic, an antiepileptic drug, an antipsychotic drug, an antidepressant and anti-anxiety drug, an antiparkinsonian drug, an antimanic drug, an antidementia drug, a sleeping drug, a sedative drug, an antispasm drug, a muscle relaxant, an autonomic drug, a cerebral circulation and metabolism improver, a cardiotonic drug, an antianginal drug, a drug for hypertension and arrhythmias, a vasodilator, a depressor, a vasopressor, a diuretic, a respiratory stimulant, an antitussive and expectorant drug, a bronchodilator, an asthma and nasal allergy medication, a bronchial dilator, a cold medication, an antiemetic, an antacid, and an anti-ulcerogenic drug.

## Patentansprüche

1. Laminierte Klebefolie für die Haut, umfassend eine Hauthaftschicht, eine Zwischenschicht, eine schützende Haftschicht und einen hochdehnbaren Träger, die in dieser Reihenfolge laminiert sind,
wobei die Hauthaftschicht ein Acrylklebemittel oder ein Urethanklebemittel enthält, wobei der hochdehnbare Träger aus einem Polyurethanfilm, einem Ethylenvinylacetatfilm, einem Urethanvliesstoff oder einem Polyestervliesstoff besteht,
wobei die schützende Haftschicht ein Kautschukklebemittel enthält,
wobei die Zwischenschicht aus einer Polyurethanfolie oder einer Ethylenvinylacetatfolie besteht,
wobei die laminierte Klebefolie eine Wasserdampfdurchlässigkeitsrate von 200 g/m²·24 h oder weniger aufweist.

2. Laminierte Klebefolie nach Anspruch 1 mit einem Zugelastizitätsmodul von 30 N oder weniger.

3. Laminierte Klebefolie nach Anspruch 1 oder 2, wobei die Hauthaftschicht eine Dicke von 20 bis 80 µm aufweist.

4. Laminierte Klebefolie nach einem der Ansprüche 1 bis 3, wobei der hochdehnbare Träger eine Dicke von 5 bis 60 µm aufweist.

5. Laminierte Klebefolie nach einem der Ansprüche 1 bis 4, umfassend eine Trennfolie auf einer Oberfläche der Hauthaftschicht.

6. Laminierte Klebefolie nach einem der Ansprüche 1 bis 5, wobei die Hauthaftschicht ein Arzneimittel oder eine wertvolle Substanz enthält,
wobei die wertvolle Substanz ausgewählt ist aus der Gruppe bestehend aus einer aufhellenden Komponente, einer Anti-Falten-Komponente, einer entzündungshemmenden Komponente, einer durchblutungsfördernden Komponente, einer antibakteriellen Komponente, einer juckreizstillenden Komponente, verschiedenen Vitaminen und Derivaten davon, einer antioxidativen Komponente, einer antiallergischen Komponente und einem Parfüm.

7. Laminierte Klebefolie nach Anspruch 6, wobei das Arzneimittel ausgewählt ist aus der Gruppe bestehend aus einem fiebersenkenden und entzündungshemmenden Analgetikum, einem Antiepileptikum, einem Antipsychotikum, einem Antidepressivum und angstlösenden Arzneimittel, einem Antiparkinson-Arzneimittel, einem Antimanie-Arzneimittel, einem Antidementivum, einem Schlafmittel, einem Sedativum, einem krampflösenden Arzneimittel, einem Muskelrelaxans, einem autonomen Arzneimittel, einem Mittel zur Verbesserung der Hirndurchblutung und des Stoffwechsels, einem kardiotonischen Arzneimittel, einem Antianginalmittel, einem Arzneimittel gegen Bluthochdruck und Herzrhythmusstörungen, einem Vasodilatator, einem Depressor, einem Vasopressor, einem Antitussivum und Expectorans, einem Bronchodilatator, einem Arzneimittel gegen Asthma und Nasenallergien, einem bronchienerweiternden Arzneimittel, einem Erkältungsmittel, einem Antiemetikum, ein Antazidum und einem Antiulzerogen.

## Revendications

1. Feuille adhésive stratifiée pour la peau comportant une couche d'adhérence à la peau, une couche intermédiaire, une couche d'adhérence protectrice et un support fortement étirable, stratifiés dans cet ordre,
dans laquelle la couche d'adhérence à la peau contient un agent adhésif acrylique ou un agent adhésif à base d'uréthane,
dans laquelle le support fortement étirable est constitué d'un film de polyuréthane, d'un film d'éthylène-acétate de vinyle, d'un tissu non tissé d'uréthane ou d'un tissu non tissé de polyester,
dans laquelle la couche d'adhérence protectrice contient un agent adhésif en caoutchouc,
dans laquelle la couche intermédiaire est constituée d'un film de polyuréthane ou d'un film d'éthylène-acétate de vinyle,
la feuille adhésive stratifiée ayant une vitesse de perméation à la vapeur d'eau de 200 g/m^{2•}24 h ou moins.

2. Feuille adhésive stratifiée selon la revendication 1, ayant un module d'élasticité en traction de 30 N ou moins.

3. Feuille adhésive stratifiée selon la revendication 1 ou 2, dans laquelle la couche d'adhérence à la peau a une épaisseur de 20 à 80 µm.

4. Feuille adhésive stratifiée selon l'une quelconque des revendications 1 à 3, dans laquelle le support fortement étirable a une épaisseur de 5 à 60 µm.

5. Feuille adhésive stratifiée selon l'une quelconque des revendications 1 à 4, comportant une feuille de décollement sur une surface de la couche d'adhérence à la peau.

6. Feuille adhésive stratifiée selon l'une quelconque des revendications 1 à 5, dans laquelle la couche d'adhérence à la peau contient un médicament ou une substance valorisable,
dans laquelle la substance valorisable est choisie parmi le groupe constitué d'un composant de blanchiment, d'un composant antirides, d'un composant anti-inflammatoire, d'un composant favorisant la circulation sanguine, d'un composant antibactérien, d'un composant anti-gravure, de diverses vitamines et de dérivés de celles-ci, d'un composant antioxydant, d'un composant anti-allergénique et d'un parfum.

7. Feuille adhésive stratifiée selon la revendication 6, dans laquelle le médicament est choisi parmi le groupe constitué d'un analgésique antipyrétique et anti-inflammatoire, d'un médicament anti-épileptique, d'un médicament antipsychotique, d'un médicament antidépresseur et anxiolytique, d'un médicament antiparkinsonien, d'un médicament antimaniaque, d'un médicament antidémence, d'un médicament somnifère, d'un médicament sédatif, d'un médicament antispasmodique, d'un relaxant musculaire, d'un médicament autonome, d'un agent améliorant la circulation et le métabolisme cérébraux, d'un médicament cardiotonique, d'un médicament anti-angineux, d'un médicament pour l'hypertension et les arythmies, d'un vasodilatateur, d'un dépresseur, d'un vasopresseur, d'un diurétique, d'un stimulant respiratoire, d'un médicament antitussif et expectorant, d'un bronchodilatateur, d'une médication contre l'asthme et l'allergie nasale, d'un dilatateur bronchique, d'une médication contre le rhume, d'un antiémétique, d'un antiacide et d'un médicament anti-ulcérogène.
